# EUROPEAN PATENT APPLICATION

(11) **EP 3 644 161 A1**
(43) Date of publication of application: **29.04.2020**
(21) Application number: 19188342.0
(22) Date of filing: 25.07.2019
(51) Int. Cl.: G06F 3/01, G16H 20/30

(54) **REAL-TIME AUGMENTED REALITY ACTIVITY FEEDBACK**

(30) Priority: 06.08.2018 US 201816055973
(71) Applicant: Motorola Mobility LLC, Chicago, IL 60654 (US)
(72) Inventor: VISSA, Sudhir, Bensenville, IL Illinois 60106 (US); MADHUSUDHANA, Nikhil, Chicago, IL Illinois 60657 (US); TYAGI, Vivek, Chicago, IL Illinois 60654 (US)
(74) Representative: Openshaw & Co.

(57) **Abstract**

In aspects of real-time augmented reality (AR) activity feedback, a system includes wearable sensors to monitor a musculoskeletal state of a person who engages in an activity. A sensor device hub includes an activity comparison module that is implemented to receive sensor information from the wearable sensors as the person engages in a session of the activity. The activity comparison module can determine activity data from the received sensor information, including a first form of how the person is performing the activity. The activity comparison module of the sensor device hub is also implemented to compare the first form of the person performing the activity to a representative form of the activity, and initiate a real-time comparison feedback that is an indication of how the first form of the person performing the activity compares to the representative form of the activity.

## Description

### BACKGROUND

Conventionally, activity trackers or fitness monitors may be used to track the number of steps taken, the distance traveled, elevation gained, and calories burned by a person while they engage in an activity. In addition some trackers and monitors may be able to monitor a person's heart rate and connect to a smartphone or tablet using a software application to keep track of targets, as well as record tracked information. However, traditional activity trackers and fitness monitors do not monitor multiple components such as muscle activity, skeletal alignment, pressure, and body tilt and rotation to aid in determining a person's musculoskeletal state. Typically, conventional trackers and monitors simply provide historical data, for example, the distance traveled while jogging, or elevation gained during a hike.

### BRIEF DESCRIPTION OF THE DRAWINGS

Aspects of real-time augmented reality activity feedback are described with reference to the following Figures. The same numbers may be used throughout to reference similar features and components that are shown in the Figures:
FIG. 1 illustrates an example environment in which aspects of real-time augmented reality activity feedback can be implemented.
FIG. 2 illustrates an example device for real-time augmented reality activity feedback in accordance with one or more implementations described herein.
FIG. 3 illustrates an example method of real-time augmented reality activity feedback in accordance with one or more implementations described herein.
FIG. 4 illustrates an example method of real-time augmented reality activity feedback in accordance with one or more implementations described herein.
FIG. 5 illustrates various components of an example device that can implement aspects of real-time augmented reality activity feedback.

### DETAILED DESCRIPTION

Aspects of real-time augmented reality (AR) activity feedback are described, and features allow a person who performs an activity to be provided with visual and/or audible instructions as feedback, as well as any form of haptic feedback, pertaining to performance of the activity by the person in real-time. Generally, a system of multiple wearable sensors can record the musculoskeletal and cardiovascular state of the person while performing the activity, and sensor data from the multiple wearable sensors can be communicatively linked to a sensor device hub. Any one of the wearable sensors may be implemented as the sensor device hub, or alternatively, the sensor device hub may be implemented as a device separate from the wearable sensors. The sensor device hub is designed to use the wearable sensor information to determine a form of the person performing the activity, compare the form of the person performing the activity to a representative form of the activity, and provide the comparison as real-time feedback in the form of visual, audible, and/or haptic feedback.

In implementations, the sensor device hub can include a radio frequency identification (RFID) reader, and the wearable sensors may be equipped with RFID tags or can be implemented as wireless sensors. For example, the sensor device hub may be implemented as a mobile phone that includes the RFID reader either as an integrated component or as an attachment that operates with the mobile phone. Generally, a person using the wearable sensors equipped with the RFID tags will perform an activity, such as a type of exercise or physical therapy. The sensor device hub equipped with the RFID reader can then read the sensor data from the RFID tags associated with each wearable sensor, where the sensor data includes information corresponding to the musculoskeletal and cardiovascular state of the person. Similarly, the sensor hub device may be equipped with wireless sensor readers or radio systems that communicate with the wireless sensors worn by the person who performs the activity to obtain the sensor data from the wireless sensors.

In aspects of real-time augmented reality activity feedback, the sensor device hub can also implement an activity comparison module that is designed to compare the form of a person performing an activity, determined from the sensor data, to a representative form of the activity being performed. The activity comparison module can then initiate a real-time activity feedback as an indication of how to improve the form of the person who is performing the activity. The sensor data, read by the RFID reader of the sensor device hub from the RFID tags associated with the wearable sensors, provides the necessary information to determine how the person is performing the exercise, such as muscle activity, skeletal positioning, oxygen intake, and such. A form of the person performing the activity is determined and compared to a representative form of the activity, where generally, an example representative form is a correct form of the activity. A real-time comparison feedback is initiated that indicates to the person performing the activity, how to improve or correct their form. The real-time comparison feedback may be in any form of visual, audible, and/or haptic feedback. For example, an avatar representing the person performing the activity may be provided as visual feedback as well as audible instructions for how the person may improve their form while performing the activity.

For example, a person may be exercising in a gym and performing a particular activity, such as doing push-ups while wearing musculoskeletal and cardiovascular sensors equipped with RFID tags. As the person is performing the push-ups, the RFID tags store the sensor data corresponding to a musculoskeletal state of the person. The person may also have a mobile phone, implemented as a sensor device hub equipped with an RFID reader, which reads the sensor data. The form of the person performing the activity is determined from the sensor data and compared to a correct form of a push-up. As the person is doing the push-up, real-time feedback is provided for the person via the mobile device. Alternatively or in addition, the real-time feedback may be provided as any form of haptic feedback, such as directly from a wireless sensor with a vibration component that generates a haptic indication as direct feedback to the person. Generally, the real-time feedback can be generated and/or provided from any of the various components of the system, to include the wireless sensors and the mobile device. The real-time feedback can provide information to the person letting them know if they are doing the push-up correctly, and if not, what they need to do to correct their form. Because the feedback is in real-time, the person is able to monitor the changes they make while doing the push-up to continually improve their form from one push-up to the next push-up.

As noted above, the real-time comparison feedback provided may be any form of visual, audible, and/or haptic feedback. For example, audible instructions on how to improve the push-up form by the person may be played through the speakers on the mobile device. Alternately or in addition, a visual indication may be provided on a display screen of the mobile device. The visual indication can include graphical data and/or textual instructions on how to improve the form of the push-ups being performed by the person. Additionally or alternatively, an avatar can be displayed that represents the person performing the push-up and/or an avatar is displayed of push-ups being performed correctly for comparison.

In aspects of real-time augmented reality activity feedback, the sensor device hub can also be implemented with location enabled tracking. The location enabled tracking can be used to track an activity route of a session of an activity performed by a person in order for the sensor device hub to provide a same route to the person for another session of the activity. For example, when a person goes for a run or a jog as an activity, the sensor device hub implemented with location enabled tracking can track the route and distance that the person runs. At a later time, the sensor device hub can provide the person with the same route and distance as the visual, audible, and/or haptic feedback for comparison with another running session.

While features and concepts of real-time augmented reality activity feedback can be implemented in any number of different devices, systems, environments, and/or configurations, aspects of real-time augmented reality activity feedback are described in the context of the following example devices, systems, and methods.

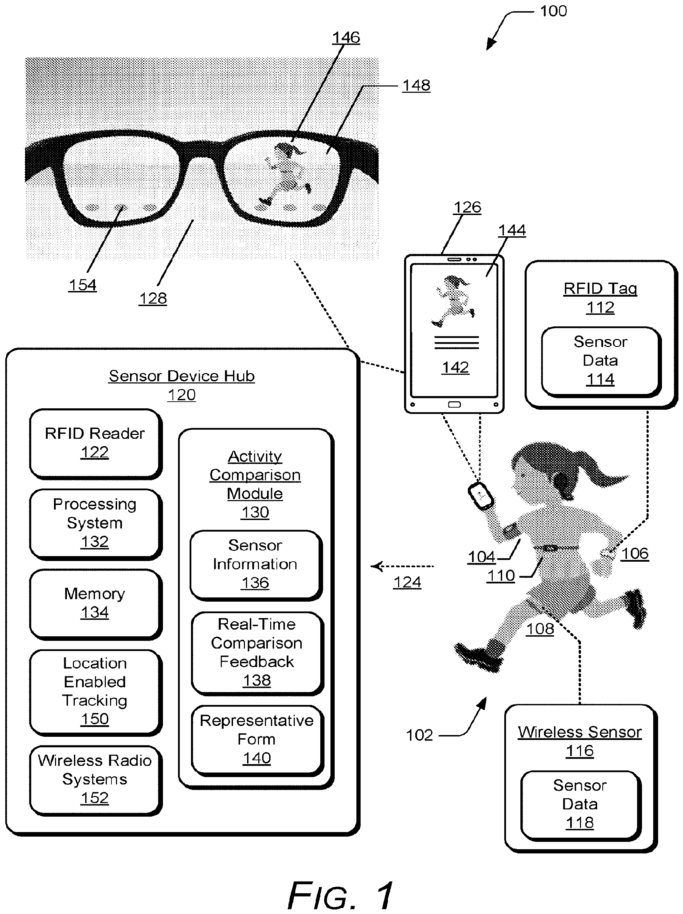

FIG. 1 illustrates an example environment 100 in which aspects of real-time augmented reality activity feedback can be implemented. The example environment 100 may be implemented in any real environment, outdoors or indoors (e.g. on the beach or inside a health club), where a person may wish to perform any kind of physical activity, such as exercise, physical therapy, amputee rehabilitation, repetitive lifting associated with a job, and the like. The example environment 100 includes a person 102 with multiple wearable sensors while performing an activity, in this example jogging. The wearable sensors may be implemented as, but are not limited to, any kind of musculoskeletal sensors (*e.g.*, an arm sensor 104, a wrist-located sensor 106, and a leg sensor 108), as well as cardiovascular sensors (*e.g.*, a cardio sensor 110) intended to acquire data related to the activity of the person. For example, the wearable sensors acquire the data related to muscle activity, skeletal position and alignment, body movement and pressure, heart rate, oxygen intake, and any other sensor data related to the body position and condition of the person in real-time. Although shown separately, the sensors may alternately or additionally be implemented in other configurations such as a single full body suit or several articles of clothing equipped with a sensor or sensors. In addition, to aid in amputee rehabilitation, sensors may be built into prosthetics in order to implement real-time augmented reality activity feedback.

The wearable sensors can be equipped with a wireless radio-based tag such as a radio frequency identification (RFID) tag. Generally, the RFID tags are small electronic tags that record sensor data and other sensor information in memory. For example, the wrist-located sensor 106 is implemented with an RFID tag 112, which includes a memory that stores sensor data 114 as acquired from the wearable sensor 106 that the RFID tag is associated with. Similarly, the other wearable sensors may each be equipped with a respective RFID tag for storing data acquired by the associated wearable sensor. Although described as RFID tags, the wireless-radio based tags (*e.g.*, RFID tag 112) can be implemented for various different radio-based, wireless tag signaling, such as with LTE, Near Field Communication (NFC), Real-time Locating System (RTLS), Bluetooth™ devices, and any other type of wireless sensor. For example, the wearable leg sensor 108 is implemented as a wireless sensor 116, which includes a memory that stores sensor data 118 as acquired from the wearable leg sensor 108.

In addition, the example environment 100 includes a sensor device hub 120 equipped with an RFID reader 122 (or other type of wireless sensor reader) implemented to transmit interrogation signals as broadcast messages requesting the RFID tags and/or other wireless sensors associated with the wearable sensors to return the sensor data 114, 118 stored in memory of the RFID tags and/or other wireless sensors. An RFID tag and/or other wireless sensor can receive an interrogation signal from the RFID reader 122 or other type of wireless sensor reader, and then wirelessly communicate the sensor data at 124 back to the RFID reader and/or other wireless sensor reader of the sensor device hub 120. Generally, the sensor device hub 120 may be implemented as any one or more of the wearable sensors and/or devices described in this example environment. For example, the sensor device hub 120 can be implemented or designated as the wireless sensor 116, as a mobile phone 126 that is carried by the person 102, or as augmented reality (AR) glasses 128 that may be worn by a person. Notably, the augmented reality (AR) glasses 128 can be used individually or in combination with the mobile phone 126 to implement the sensor device hub. The terms "person" and "user" are generally used herein interchangeably, where the person 102 with the mobile phone 126 is also the user of the mobile phone in the example environment 100.

In this example, the sensor device hub 120 includes an activity comparison module 130 that implements features of the described real-time augmented reality activity feedback. In implementations, the activity comparison module 130 may include independent processing, memory, and logic components functioning as a computing and/or electronic device integrated with the sensor device hub 120. Alternatively or in addition, the activity comparison module 130 can be implemented as a software application, such as computer-executable software instructions that are executable with a processor or processing system 132 of the sensor device hub. As a software application, the activity comparison module 130 can be stored on computer-readable storage memory 134, or with any suitable memory device or electronic data storage implemented with the sensor device hub.

The activity comparison module 130 that is implemented by the sensor device hub 120 can receive the sensor data at 124 stored in memory of a wireless sensor and/or an RFID tag of each wearable sensor, such as the sensor data 114 received from the wearable sensor 106 and the sensor data 118 received from the wearable sensor 108. The activity comparison module 130 receives the sensor data 114, 118 as the sensor information 136, and from the sensor information, the activity comparison module can determine the form of the activity performed by the person 102 who is exercising, in this example jogging or running. A real-time comparison feedback 138 can then be determined by the activity comparison module 130 comparing the form of the person performing the activity (*e.g.*, exercising) to a representative form 140 of the activity (*e.g.*, exercise) being performed, and then providing an indication of how the form of the person exercising compares to the representative form. For example, the real-time comparison feedback 138 can be determined by comparing the jogging form of the person 102 determined from the sensor information 136 with a representative form 140, where the representative form may be a correct or ideal form of jogging or running.

An indication of how the jogging form of the person 102 compares to the correct jogging form can be provided to the user, in this example as a visual indication of an avatar and instructions 142 shown on the display screen 144 of the mobile phone 126 implemented as the sensor device hub. In an alternate example, the AR glasses 128 display an avatar 146 of the representative form for performing the activity on lenses 148 of the glasses such that the user can see the avatar 146 overplayed on the real environment, in this example on a beach where the user is jogging.

The sensor device hub 120 may also be equipped with location enabled tracking 150 implemented to track an activity route of a person engaging in an activity that results in the person covering a specific route and distance, such as in this example jogging. The route of the person performing the activity can be tracked and saved such that the same route can be provided to the person at a later time during a subsequent session of the activity as the representative form 140. The AR glasses 128 show a route from a previous session of jogging being provided for viewing by the person as an AR path 154 overlaid on the real environment in which the person has jogged before and is currently jogging. Alternately or in addition, the avatar 146 may represent the person jogging the route depicted by the AR path 154 during a previous jogging session. In this way, the person may jog with, or compete against their own previous jogging session. Notably, the avatar 146 may represent the previous best performance of the person on the route depicted by the AR path 154, allowing the person to try and beat their previous best performance. Additionally, the representative form 140 may be the avatar 146 representing the jogging performance of another person along the route, such as a friend whose activity performance the person 102 wants to compete against.

In addition to the RFID implementation, the sensor device hub 120 can include various, different wireless radio systems 152, such as for Wi-Fi, Bluetooth™, Mobile Broadband, LTE, Near Field Communication (NFC), Real-time Locating System (RTLS), or any other wireless radio system or format for communication via respective wireless networks. Generally, the sensor device hub 120 implements the wireless radio systems 152 that each include a radio device, antenna, and chipset implemented for cellular, wireless, and/or other network communication with other devices, networks, and services. A wireless radio system 152 can be configured to implement any suitable communication protocol or standard. Although features and aspects of real-time augmented reality activity feedback are described and illustrated in the context of RFID tags, the described features and aspects can be implemented with various radio-based, wireless tag signaling, such as with LTE, NFC, RTLS, Bluetooth™ devices, and the like.

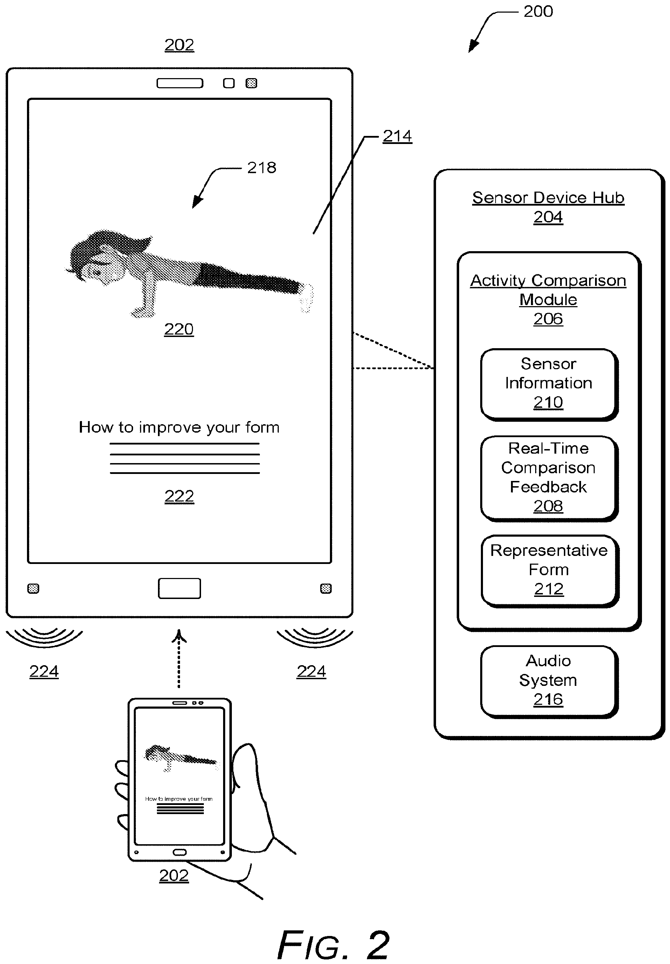

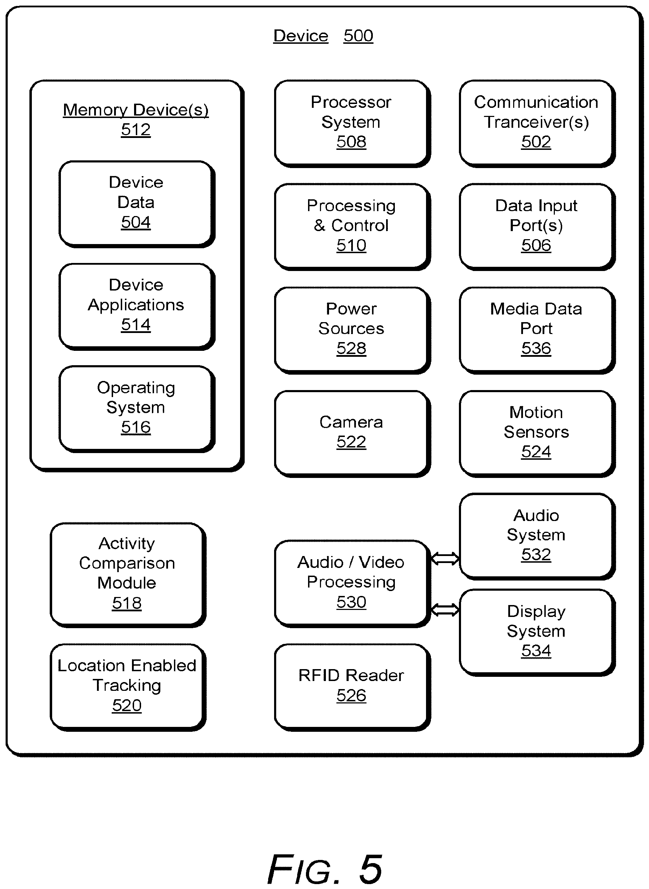

FIG. 2 illustrates an example device 200 in which aspects of real-time augmented reality activity feedback can be implemented. The example device 200 may be any type of electronic device, such as a mobile device 202, the augmented reality (AR) glasses 128, a tablet device, a wearable device, a desktop computing device, or any other type of electronic and/or computing device described with reference to FIG. 5 that is implemented as a sensor device hub 204. Alternately or in addition, the sensor device hub 204 may be implemented as a cloud-based service that the mobile device 202 can communicate with via a communication network. The sensor device hub 204 is implemented with an activity comparison module 206, similar to the activity comparison module 130 that is shown and described with reference to FIG. 1, implemented to provide a real-time comparison feedback 208 of activity performances. As noted above, activity data including a form of how a person is performing an activity can be determined from sensor information 210. A comparison of the form of the person performing the activity to a representative form 212 of the activity being performed is determined, and the real-time comparison feedback 208 is generated as an indication of how the form of the person performing the activity compares to the representative form 212 of the activity being performed.

The example mobile device 202 implemented as the sensor device hub 204 can also include an integrated display 214 and/or an audio system 216 for providing visual feedback, audible feedback, and/or haptic feedback as the real-time comparison feedback 208. In this example, a visual feedback 218 is displayed as an avatar 220 of the person performing an activity, and the visual feedback 218 may include textual instructions 222 detailing steps for the person to improve their form of performing the activity. Additionally, the activity comparison module 206 can initiate audible feedback 224 as instructions via the audio system 216 of the mobile device, indicating how the user can improve the form of performing the activity. Alternately or in addition, the mobile device 202 may be implemented as the sensor device hub, with visual feedback provided by a pair of augmented reality (AR) glasses 128 such as those shown in FIG. 1. Alternate implementations may also be considered, such as the mobile device 202 being a mobile phone of a user and implemented as the sensor device hub in communication with other devices to provide the visual and/or audible feedback, such as via a wall mounted display device in a health club or physical therapy facility.

In other implementations of real-time augmented reality activity feedback, the avatar 220 shown as the visual feedback 218 on the integrated display 214 of the mobile device 202 may be the representative form 212 of the activity. The representative form 212 of the activity may be the correct form of the activity being performed, or alternatively, may represent the person performing the activity during a previous session of the activity, or may be a representation of an additional person performing the activity, such as a personal trainer, a group exercise instructor, or a physical therapist. Additionally, two avatars may be shown as the visual feedback 218, with one avatar representing the person performing the activity and the second avatar being the representation of the representative form 212 of the activity being performed.

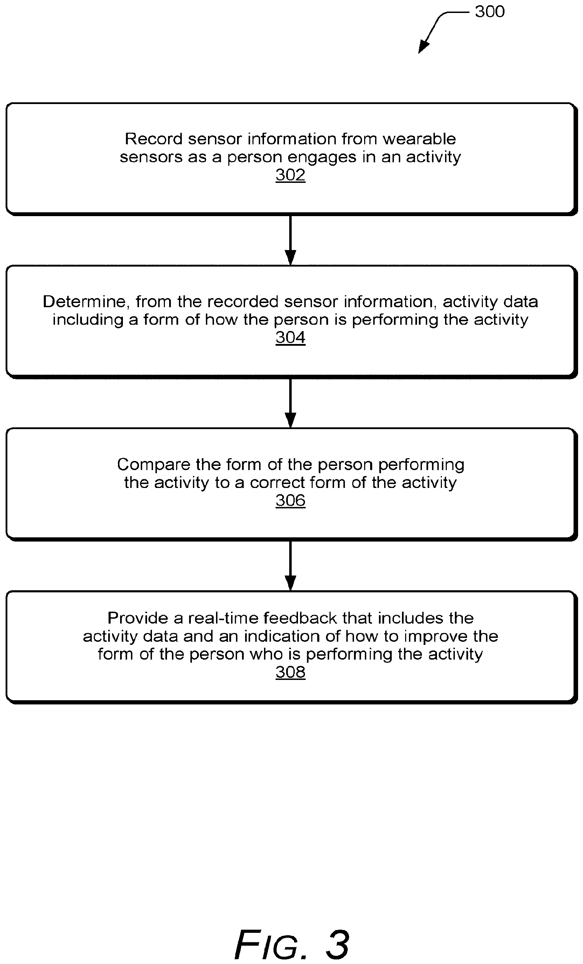

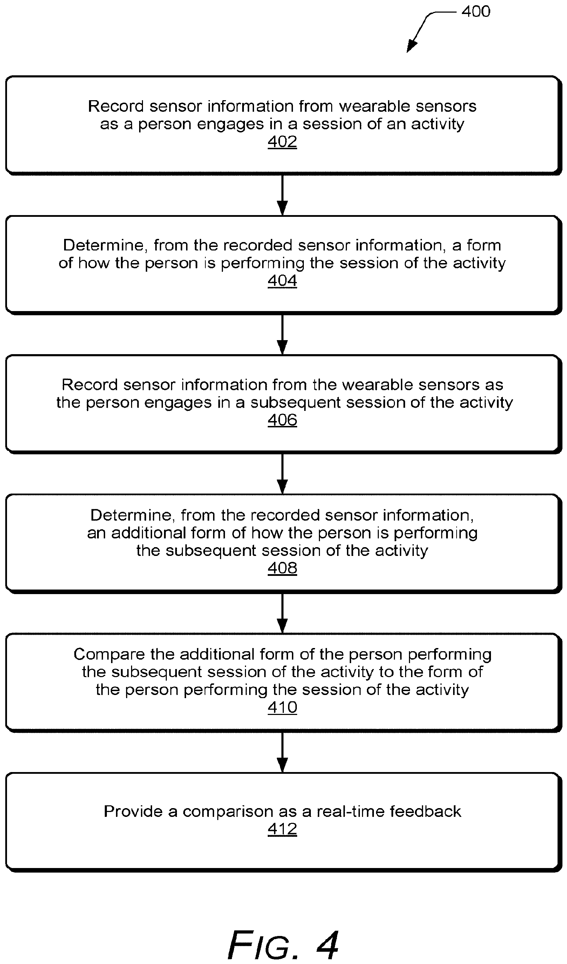

Example methods 300 and 400 are described with reference to respective FIGs. 3 and 4 in accordance with implementations of real-time augmented reality activity feedback. Generally, any services, components, modules, methods, and/or operations described herein can be implemented using software, firmware, hardware (*e.g.*, fixed logic circuitry), manual processing, or any combination thereof. Some operations of the example methods may be described in the general context of executable instructions stored on computer-readable storage memory that is local and/or remote to a computer processing system, and implementations can include software applications, programs, functions, and the like. Alternatively or in addition, any of the functionality described herein can be performed, at least in part, by one or more hardware logic components, such as, and without limitation, Field-programmable Gate Arrays (FPGAs), Application-specific Integrated Circuits (ASICs), Application-specific Standard Products (ASSPs), System-on-a-chip systems (SoCs), Complex Programmable Logic Devices (CPLDs), and the like.

FIG. 3 illustrates example method(s) 300 of real-time augmented reality activity feedback as described herein, and the method is generally described with reference to a sensor device hub implemented for communication with wireless tags that correspond to wearable sensors. The order in which the method is described is not intended to be construed as a limitation, and any number or combination of the described method operations can be performed in any order to perform a method, or an alternate method.

At 302, sensor information is recorded from wearable sensors as a person engages in an activity. For example, the activity comparison module 130 initiates a scan from the sensor device hub 120 with the RFID reader 122 of the wireless-enabled devices (*e.g.*, the RFID tag 112 of the wearable sensor 106) and/or with other types of wireless sensor readers of the wireless sensors (*e.g.*, the wireless sensor 116) associated with the multiple wearable sensors that are worn by the person 102 who is performing an activity. The sensor data 114, 118, obtained while the person is engaged in the activity, is read by the RFID reader 122 and/or other type of wireless sensor reader (*e.g.*, a wireless radio system 152), and recorded as the sensor information 136 of the activity comparison module 130. The wearable sensors can be any kind of musculoskeletal or cardiovascular sensors used to determine the muscle activity, skeletal position and alignment, body movement, pressure on surfaces of the body or joints of the person, heart rate, oxygen intake, and any other sensor data related to the person performing the activity.

At 304, activity data including a form of how the person is performing the activity is determined from the recorded sensor information. For example, the activity comparison module 130 that is implemented by the sensor device hub 120 determines the form of how the person 102 is performing the activity using the sensor information 136 obtained from the multiple wearable sensors, such as any kind of musculoskeletal sensors (*e.g.*, the arm sensor 104, the wrist-located sensor 106, and the leg sensor 108), as well as cardiovascular sensors (*e.g.*, the cardio sensor 110) intended to acquire data related to the activity of the person.

At 306, the form of the person performing the activity is compared to a correct form of the activity. For example, the activity comparison module 130 compares the form of the person 102 who is performing the activity with the representative form 140 of the activity, where the representative form 140 can be a correct form of how to perform the activity. In implementations, the activity comparison module 130 uses the sensor information 136 that is recorded as the sensor data 114, 118 from the wearable sensors worn by the person while performing the activity.

At 308, a real-time feedback is provided that includes the activity data and an indication of how to improve the form of the person who is performing the activity. For example, the activity comparison module 130 initiates providing a real-time feed-back that indicate to the person 102 how to improve their form while performing the activity. The real-time feedback is provided as haptic feedback, as the visual feedback 218, and/or as the audible feedback 224. The visual feedback 218 may include a graphical representation of the activity data comparing it to a representative form that is the correct form for performing the activity, and the visual feedback can indicate changes the person needs to make in order to improve their form. The graphical representation may also include written instructions of steps to take to improve the person's form, such as the textual instructions 222 shown in FIG. 2. Alternatively or in addition, the visual feedback 218 may include an avatar, such as the avatar 220 that represents the person performing the activity and the person can see their form and make necessary adjustments for improvement. The audible feedback 224 can include audible instructions for improving the form of the person performing the activity.

FIG. 4 illustrates example method(s) 400 of real-time augmented reality activity feedback as described herein, and the method is generally described with reference to the activity comparison module implemented by a sensor device hub. The order in which the method is described is not intended to be construed as a limitation, and any number or combination of the described method operations can be performed in any order to perform a method, or an alternate method.

At 402, sensor information is recorded from wearable sensors as a person engages in a session of an activity. For example, the activity comparison module 130 initiates a scan from the sensor device hub 120 with the RFID reader 122 of the wireless-enabled devices (*e.g.*, the RFID tag 112 of the wearable sensor 106) associated with the multiple wearable sensors worn by the person 102 who is engaging in a session of an activity. The sensor data 114, 118, obtained while the person is engaged in the activity, is read by the RFID reader 122 and/or other type of wireless sensor reader (*e.g.*, a wireless radio system 152), and recorded as the sensor information 136 of the activity comparison module 130. The wearable sensors can be any kind of musculoskeletal or cardiovascular sensors used to determine the muscle activity, skeletal position and alignment, body movement, pressure on surfaces of the body or joints of the person, heart rate, oxygen intake, and any other sensor data related to the person performing the activity.

At 404, activity data including a form of how the person is performing the session of the activity is determined from the recorded sensor information. For example, the activity comparison module 130 that is implemented by the sensor device hub 120 determines a form of how the person 102 is performing the session of the activity using the sensor information 136 obtained from the wearable sensors, such as any kind of musculoskeletal sensors (*e.g.*, the arm sensor 104, the wrist-located sensor 106, and the leg sensor 108), as well as cardiovascular sensors (*e.g.*, the cardio sensor 110) intended to acquire data related to the activity of the person.

At 406, sensor information is recorded from the wearable sensors as the person engages in a subsequent session of the activity. For example, the activity comparison module 130 initiates a scan from the sensor device hub 120 with the RFID reader 122 of the wireless-enabled devices (*e.g.*, the RFID tag 112 of the wearable sensor 106) associated with the multiple wearable sensors worn by the person 102 who is engaging in a subsequent session of the activity. The sensor data 114, 118, obtained while the person is engaged in the activity, is read by the RFID reader 122 and/or other type of wireless sensor reader (*e.g.*, a wireless radio system 152), and recorded as the sensor information 136 of the activity comparison module 130.

At 408, activity data including an additional form of how the person is performing the activity is determined from the recorded sensor information. For example, the activity comparison module 130 that is implemented by the sensor device hub 120 determines an additional form of how the person 102 is performing the activity using the sensor information 136 obtained from the wearable sensors.

At 410, the additional form of the person performing the activity during the subsequent session is compared to the form of the person performing the activity during a previous session. For example, the activity comparison module 130 compares the additional form of the person 102 performing the activity (as determined at 408) with the previous form of the person 102 person performing the activity (as determined at 404) based on the sensor information 136 obtained from the wearable sensors. Notably, a comparison of previous and subsequent forms of the person performing the activity can include a comparison between any number of previous activities, to include parts of previous activities and/or designated best parts of the activities.

At 412, a real-time comparison feedback is provided. For example, the activity comparison module 130 initiates providing a real-time comparison feedback as haptic feedback, as the visual feedback 218, and/or as the audible feedback 224. The haptic feedback may be implemented via the wireless sensors as haptic indications for improving the form of the person performing the activity. The visual feedback 218 may include a graphical representation comparing the activity data from the first session of the activity to the activity data from the second session of the activity, and indicating whether or not the form of the person performing the activity is improving from session to session. Alternatively or in addition, the visual feedback 218 may include an avatar, such as avatar 220, which represents the person performing the activity from the first session of activity, such that the person can see their form and make necessary adjustments for improvement as they engage in the second session of the activity. Alternately, the visual feedback may include two avatars of the person engaging in the activity for comparison, with one avatar representing the person engaging in the first session of the activity and a second avatar representing the person engaging in the second session of the activity. The audible feedback 224 can include an audible comparison that indicates an improvement of the form of the person engaging in the first and second sessions of the activity, as well as instructions on how the person can improve their form while performing the activity.

FIG. 5 illustrates various components of an example device 500 in which aspects of real-time augmented reality activity feedback can be implemented. The example device 500 can be implemented as any of the devices described with reference to the previous FIGs. 1-4, such as any type of mobile device, mobile phone, client device, wearable device, tablet, computing, communication, entertainment, gaming, media playback, and/or other type of electronic device. For example, the sensor device hubs 120 and 204, the mobile phone 126, the augmented reality (AR) glasses 128, and the mobile device 202 shown and described with reference to FIGs. 1-4 may be implemented as the example device 500. Further a wearable device may include any one or combination of a watch, armband, wristband, bracelet, glove or pair of gloves, glasses or goggles, jewelry items, clothing items, any type of footwear or headwear, wearable sensors, and/or other types of wearables, including a wearable item with integrated sensors.

The device 500 includes communication transceivers 502 that enable wired and/or wireless communication of device data 504 with other devices. The device data 504 can include any of the sensor data and information, real-time comparison feedback of activity performance, representative forms of performing an activity, audio data, and location tracking data. Additionally, the device data 504 can include any type of audio, video, and/or image data. Example communication transceivers 502 include wireless personal area network (WPAN) radios compliant with various IEEE 802.15 (Bluetooth™) standards, wireless local area network (WLAN) radios compliant with any of the various IEEE 802.11 (WiFi™) standards, wireless wide area network (WWAN) radios for cellular phone communication, wireless metropolitan area network (WMAN) radios compliant with various IEEE 802.16 (WiMAX™) standards, and wired local area network (LAN) Ethernet transceivers for network data communication.

The device 500 may also include one or more data input ports 506 via which any type of data, media content, and/or inputs can be received, such as user-selectable inputs to the device, messages, music, television content, recorded content, and any other type of audio, video, and/or image data received from any content and/or data source. The data input ports may include USB ports, coaxial cable ports, and other serial or parallel connectors (including internal connectors) for flash memory, DVDs, CDs, and the like. These data input ports may be used to couple the device to any type of components, peripherals, or accessories such as microphones and/or cameras.

The device 500 includes a processing system 508 of one or more processors (*e.g.*, any of microprocessors, controllers, and the like) and/or a processor and memory system implemented as a system-on-chip (SoC) that processes computer-executable instructions. The processor system may be implemented at least partially in hardware, which can include components of an integrated circuit or on-chip system, an application-specific integrated circuit (ASIC), a field-programmable gate array (FPGA), a complex programmable logic device (CPLD), and other implementations in silicon and/or other hardware. Alternatively or in addition, the device can be implemented with any one or combination of software, hardware, firmware, or fixed logic circuitry that is implemented in connection with processing and control circuits, which are generally identified at 510. The device 500 may further include any type of a system bus or other data and command transfer system that couples the various components within the device. A system bus can include any one or combination of different bus structures and architectures, as well as control and data lines.

The device 500 also includes computer-readable storage memory 512 (*e.g.*, memory devices) that enable data storage, such as data storage devices that can be accessed by a computing device, and that provide persistent storage of data and executable instructions (*e.g.*, software applications, programs, functions, and the like). Examples of the computer-readable storage memory 512 include volatile memory and non-volatile memory, fixed and removable media devices, and any suitable memory device or electronic data storage that maintains data for computing device access. The computer-readable storage memory can include various implementations of random access memory (RAM), read-only memory (ROM), flash memory, and other types of storage media in various memory device configurations. The device 500 may also include a mass storage media device.

The computer-readable storage memory 512 provides data storage mechanisms to store the device data 504, other types of information and/or data, and various device applications 514 (*e.g.*, software applications). For example, an operating system 516 can be maintained as software instructions with a memory device and executed by the processing system 508. The device applications may also include a device manager, such as any form of a control application, software application, signalprocessing and control module, code that is native to a particular device, a hardware abstraction layer for a particular device, and so on.

In this example, the device 500 includes an activity comparison module 518 that implements aspects of real-time augmented reality activity feedback, and may be implemented with hardware components and/or in software as one of the device applications 514, such as when the device 500 is implemented as the mobile phone 126 or the mobile device 202 described with reference to FIGs. 1-4. Examples of the activity comparison module 518 include the activity comparison module 130 that is implemented as a software application and/or as hardware components in the sensor device hub 120, and the activity comparison module 206 that is implemented as a software application and/or as hardware components in the sensor device hub 204. In implementations, the activity comparison module 518 may include independent processing, memory, and logic components as a computing and/or electronic device integrated with the device 500.

In this example, the device 500 also includes location enabled tracking 520 that implements aspects of real-time augmented reality activity feedback, and may be implemented with hardware components and/or in software as one of the device applications 514, such as when the device 500 is implemented as the sensor device hub 120 described with reference to FIGs. 1-4. The example device 500 also includes a camera 522 and motion sensors 524. The motion sensors 524 can be implemented with various sensors, such as a gyroscope, an accelerometer, and/or other types of motion sensors to sense motion of the device. The various motion sensors 524 may also be implemented as components of an inertial measurement unit (IMU) in the device.

The device 500 also includes a radio-frequency identification (RFID) reader 526 that is implemented to interrogate RFID tags for identifying data and receive identification responses from the RFID tags. An example of the RFID reader 526 is the RFID reader 122 that is implemented as an integrated component of the sensor device hub 120 or as an attachment that operates with the sensor device hub. In implementations, an RFID reader as an attachment to the device 500 has an external power source. The device 500 can also include one or more power sources 528, such as when the device is implemented as a mobile device. The power sources may include a charging and/or power system, and can be implemented as a flexible strip battery, a rechargeable battery, a charged super-capacitor, and/or any other type of active or passive power source.

The device 500 also includes an audio and/or video processing system 530 that generates audio data for an audio system 532 and/or generates display data for a display system 534. The audio system and/or the display system may include any devices that process, display, and/or otherwise render audio, video, display, and/or image data. Display data and audio signals can be communicated to an audio component and/or to a display component via an RF (radio frequency) link, S-video link, HDMI (high-definition multimedia interface), composite video link, component video link, DVI (digital video interface), analog audio connection, or other similar communication link, such as media data port 536. In implementations, the audio system and/or the display system are integrated components of the example device. Alternatively, the audio system and/or the display system are external, peripheral components to the example device.

Although implementations of real-time augmented reality activity feedback have been described in language specific to features and/or methods, the subject of the appended claims is not necessarily limited to the specific features or methods described. Rather, the features and methods are disclosed as example implementations of real-time augmented reality activity feedback, and other equivalent features and methods are intended to be within the scope of the appended claims. Further, various different examples are described and it is to be appreciated that each described example can be implemented independently or in connection with one or more other described examples. Additional aspects of the techniques, features, and/or methods discussed herein relate to one or more of the following:

A method for real-time augmented reality (AR) activity feedback, the method comprising: recording sensor information from wearable sensors as a person engages in an activity; determining, from the recorded sensor information, activity data including a form of how the person is performing the activity; comparing the form of the person performing the activity to a correct form of performing the activity; and providing a real-time feedback that includes the activity data and an indication of how to improve the form of the person who is performing the activity.

Alternatively or in addition to the above described method, any one or combination of: the real-time feedback is provided as at least one of a visual feedback, an audible feedback, and a haptic feedback. The visual feedback includes a graphical representation of the activity data. The visual feedback includes at least one of an avatar that represents the person performing the activity, or the avatar for said comparing the form of the person performing the activity to the correct form of performing the activity. The audible feedback includes audible instructions for improving the form of the person performing the activity. The haptic feedback includes haptic indications for improving the form of the person performing the activity.

A method for real-time augmented reality (AR) activity feedback, the method comprising: recording sensor information from wearable sensors as a person engages in a session of an activity; determining, from the recorded sensor information, a form of how the person is performing the session of the activity; recording sensor information from the wearable sensors as the person engages in a subsequent session of the activity; determining, from the recorded sensor information, an additional form of how the person is performing the subsequent session of the activity; comparing the additional form of the person performing the subsequent session of the activity to the form of the person performing the session of the activity; and providing a comparison as a real-time feedback.

Alternatively or in addition to the above described method, any one or combination of: providing the comparison includes providing an audible feedback indicating whether the subsequent session of the activity performed by the person is an improvement over the session of the activity. Providing the comparison includes displaying a first avatar that represents the person performing the session of the activity approximately synchronous with a second avatar that represents the person performing the subsequent session of the activity.

A system for real-time augmented reality (AR) activity feedback, the system comprising: wearable sensors to monitor a musculoskeletal state of a person who engages in an activity; and a sensor device hub comprising an activity comparison module configured to: receive sensor information from the wearable sensors as the person engages in a session of the activity; determine, from the received sensor information, activity data including a first form of how the person is performing the activity; compare the first form of the person performing the activity to a representative form of the activity; and initiate a real-time comparison feedback that is an indication of how the first form of the person performing the activity compares to the representative form of the activity.

Alternatively or in addition to the above described system, any one or combination of: the wearable sensors include cardiovascular sensors to monitor a cardiovascular state of the person who engages in the activity. The sensor device hub comprises an RFID reader, and the wearable sensors include RFID tags implemented to record the musculoskeletal state and the cardiovascular state of the person performing the activity. The representative form of the activity is a correct form of the activity. The representative form of the activity is of the person performing the activity during a previous session of the activity. The representative form of the activity is of at least one additional person performing the activity. The sensor device hub is a mobile device configured to provide the real-time comparison feedback as at least one of a visual feedback, an audible feedback, and a haptic feedback. The sensor device hub is a pair of glasses equipped with an augmented reality (AR) screen to display an overlay of an avatar that represents the person performing the activity in a real environment in which the person is performing the activity. The sensor device hub comprises location enabled tracking implemented to track an activity route of a session of the activity performed by the person in order for the sensor device hub to provide a same route to the person for another session of the activity and provide the real-time comparison feedback. The representative form of the activity is a form of the person performing a previous session of the activity as an avatar of the person for competition against the previous session of the activity. The representative form of the activity is a correct form of the activity provided as an avatar correctly performing the activity for the person to compete against or keep pace with.

## Claims

1. A method for real-time augmented reality (AR) activity feedback, the method comprising:
recording sensor information from wearable sensors as a person engages in an activity;
determining, from the recorded sensor information, activity data including a form of how the person is performing the activity;
comparing the form of the person performing the activity to a correct form of performing the activity; and
providing a real-time feedback that includes the activity data and an indication of how to improve the form of the person who is performing the activity.

2. The method as recited in claim 1, wherein the real-time feedback is provided as at least one of a visual feedback, an audible feedback, and a haptic feedback.

3. The method as recited in claim 2, wherein the visual feedback includes a graphical representation of the activity data.

4. The method as recited in claim 2 or 3, wherein the visual feedback includes at least one of an avatar that represents the person performing the activity, or the avatar for said comparing the form of the person performing the activity to the correct form of performing the activity.

5. The method as recited in any of claims 2 to 4, wherein the audible feedback includes audible instructions for improving the form of the person performing the activity.

6. The method as recited in any of claims 2 to 5, wherein the haptic feedback includes haptic indications for improving the form of the person performing the activity.

7. A method for real-time augmented reality (AR) activity feedback, the method comprising:
recording sensor information from wearable sensors as a person engages in a session of an activity;
determining, from the recorded sensor information, a form of how the person is performing the session of the activity;
recording sensor information from the wearable sensors as the person engages in a subsequent session of the activity;
determining, from the recorded sensor information, an additional form of how the person is performing the subsequent session of the activity;
comparing the additional form of the person performing the subsequent session of the activity to the form of the person performing the session of the activity; and
providing a comparison as a real-time feedback.

8. The method as recited in claim 7, wherein providing the comparison includes providing an audible feedback indicating whether the subsequent session of the activity performed by the person is an improvement over the session of the activity performed by the person.

9. The method as recited in claim 7 or 8, wherein providing the comparison includes displaying a first avatar that represents the person performing the session of the activity approximately synchronous with a second avatar that represents the person performing the subsequent session of the activity.

10. A system for real-time augmented reality (AR) activity feedback, the system comprising:
wearable sensors to monitor a musculoskeletal state of a person who engages in an activity; and
a sensor device hub comprising an activity comparison module configured to:
receive sensor information from the wearable sensors as the person engages in a session of the activity;
determine, from the received sensor information, activity data including a form of how the person is performing the activity;
compare the form of the person performing the activity to a representative form of the activity; and
initiate a real-time comparison feedback that is an indication of how the form of the person performing the activity compares to the representative form of the activity.

11. The system as recited in claim 10, wherein the wearable sensors include cardiovascular sensors to monitor a cardiovascular state of the person who engages in the activity.

12. The system as recited in claim 11, wherein the sensor device hub comprises an RFID reader, and the wearable sensors include RFID tags implemented to record the musculoskeletal state and the cardiovascular state of the person performing the activity.

13. The system as recited in any of claims 10 to 12, wherein the representative form of the activity is a correct form of the activity.

14. The system as recited in any of claims 10 to 13, wherein the representative form of the activity is of the person performing the activity during a previous session of the activity.

15. The system as recited in any of claims 10 to 14, wherein the representative form of the activity is of at least one additional person performing the activity.

16. The system as recited in any of claims 10 to 15, wherein the sensor device hub is a mobile device configured to provide the real-time comparison feedback as at least one of a visual feedback, an audible feedback, and a haptic feedback.

17. The system as recited in any of claims 10 to 16, wherein the sensor device hub is a pair of glasses equipped with an augmented reality (AR) screen to display an overlay of an avatar that represents the person performing the activity in a real environment in which the person is performing the activity.

18. The system as recited in any of claims 10 to 17, wherein the sensor device hub comprises location enabled tracking implemented to track an activity route of a session of the activity performed by the person in order for the sensor device hub to provide a same route to the person for another session of the activity and provide the real-time comparison feedback.

19. The system as recited in any of claims 10 to 18, wherein the representative form of the activity is a form of the person performing a previous session of the activity as an avatar of the person for competition against the previous session of the activity.

20. The system as recited in any of claims 10 to 19, wherein the representative form of the activity is a correct form of the activity provided as an avatar correctly performing the activity for the person to compete against or keep pace with.
